# EUROPÄISCHE PATENTSCHRIFT

(11) **EP 0 659 099 B1**
(45) Veröffentlichungstag und Bekanntmachung des Hinweises auf die Patenterteilung: **31.01.1996**
(21) Anmeldenummer: 93918928.8
(22) Anmeldetag: 26.08.1993
(51) Int. Cl.: A61N 1/32

(54) **ELEKTROTHERAPEUTISCHES GERÄT**
ELECTROTHERAPY DEVICE
APPAREIL D'ELECTROTHERAPIE

(30) Priorität: 05.09.1992 DE 4229693
(43) Veröffentlichungstag der Anmeldung: 28.06.1995
(73) Patentinhaber: HANSJÜRGENS, Achim, D-76229 Karlsruhe (DE)
(72) Erfinder: HANSJÜRGENS, Achim, D-76229 Karlsruhe (DE)
(74) Vertreter: Zahn, Roland, Dipl.-Ing.
(86) Internationale Anmeldenummer: DE9300789
(87) Internationale Veröffentlichungsnummer: WO9405370

(56) Entgegenhaltungen:
- DE-A- 3 335 849
- US-A- 3 774 620
- US-A- 5 018 524
- US-A- 5 048 523

## Beschreibung

Die vorliegende Erfindung bezieht sich auf ein im Mittelfrequenzbereich zwischen 1.000Hz und 100.000Hz arbeitendes Gerät für elektrotherapeutische Anwendungen, wobei bezogen auf ein zu behandelndes Körperteil sich paarweise diametral gegenüberliegende Elektroden angelegt werden.

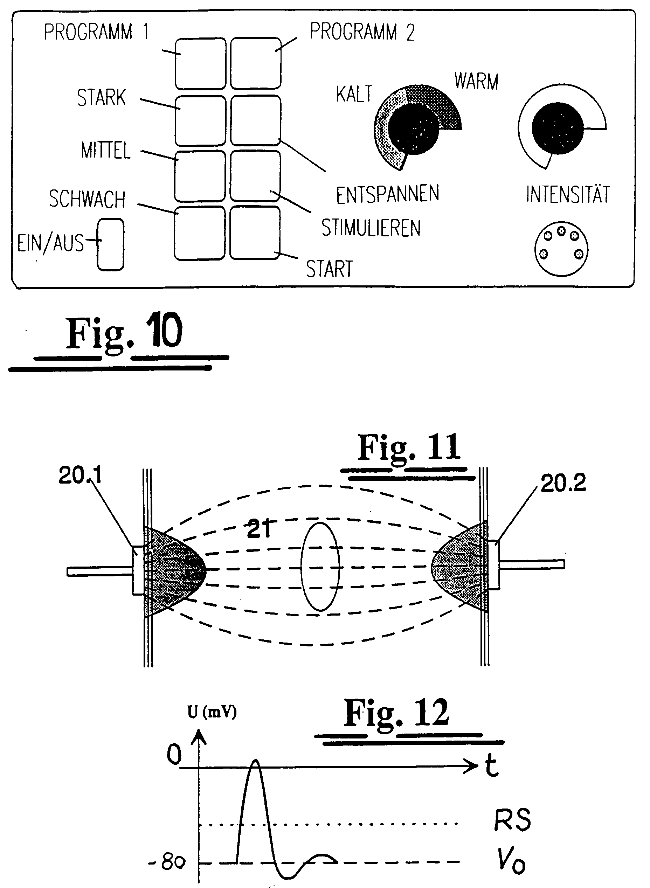

Es ist seit langem bekannt und wird insoweit auch elektrotherapeutisch genutzt, erregbare Zellen (Nerven, Muskeln und Rezeptoren von Nervenendigungen) des menschlichen Körpers durch elektrische Reize, die in Form von elektrischen Impulsen von außen zugeführt werden, zu einer elektrischen Antwort, den sogenannten Aktionspotentialen (vgl. Fig. 12), zu zwingen. Diese Aktionspotentiale sind zelleigene elektrische Impulse mit definierter Höhe und Breite für den jeweiligen Zelltyp. So ist für einen Nerv die Impulsbreite von ca. 1ms und die Höhe von ca. 80 mV - 100 mV typisch. Die Zelle greift dabei auf ihre Zellmembranspannung zurück, die in Ruhe, je nach Zelltyp, einen Wert zwischen 60 mV und 120 mV aufweist. Diese Spannung wird durch unterschiedliche Ionenkonzentrationen in den extra- und intrazellulären Räumen hervorgerufen, die durch die Zellmembran getrennt sind. Außerhalb der Zelle sind mehr positive Ionen zu finden. Definitionsgemäß wird das Potential außerhalb der Zelle auf 0V gesetzt, so daß sich in der Zelle ein negatives Potential ergibt (vgl. Fig. 12).

Im gesunden Menschen werden Aktionspotentiale vom Körper selbst erzeugt und zur Informationsübertragung und zum Auslösen von Zellvorgängen genutzt. In der Elektrotherapie werden durch gezielte Erzeugung von Aktionspotentialen (definierte Anzahl und an bestimmten Orten) therapeutische Wirkungen erzeugt.

Bei bekannten Geräten für die Elektrotherapie wird eine Vielzahl von unterschiedlichen Strom- bzw. Impulsformen verwandt. Bei dem Bestreben des Therapeuten, die am besten geeignete Elektrotherapei für die im (speziefischen) Einzelfall vorliegende Indikation auszuwählen, sollte er auf möglichst klar definierte Kriterien zurückgreifen können. Diese Kriterien ergeben sich dabei jeweils aus den Antworten auf die Fragen nach der Wirksamkeit und der Verträglichkeit der verschiedenen Stromformen.

Das Spektrum der Wirkungen umfaßt dabei z. B. die Bereiche der Schmerzlinderung, der Reizung von quergestreiften und von glatten Muskulatur, der Durchblutungsbeein- flussung, der Stoffechselbeeinflussung, der abschwellenden Mechanismen, der Entzündungshemmung und der Förderung der Regeneration (Wunden, beschleunigte Knochenheilung). Dabei sollte bei der Applikation immer versucht werden, durch richtige Wahl der Stromform die erwünschten Wirkungen im erkrankten Gebiet, entweder elektrodennah oder elektrodenfern bzw. in der Tiefe der Körpers, zu erzielen.

Bezüglich der Verträglichkeit soll sichergestellt sein, daß der Strom sowohl systemisch als auch lokal keine Schäden verursacht.

Die systemische Verträglichkeit von Strömen wird dabei hauptsächlich durch die Herzkammerflimmerschwelle und die Gefahr der Auslöäsung von epileptischen Anfällen bestimmt. Das Bedeutet, daß der therapeutische Bereich möglichst weit von diesen Schwellen entfernt sein soll. Es sind demnach Ströme vorzuziehen, bei denen diese Schwellen besonders hoch liegen.

Die lokale Verträglichkeit wird durch die Gefahr von Verbrennungen und Verätzungen, sowie durch die Schmerzschwelle bestimmt.

Demnach sind Ströme und Impulsformen vorzuziehen, bei denen die gewünschte Wirkungen zustandekommen, ohne daß der Patient irgendwelche negatieven Begleiterscheinungen verspürt.

Grundsätzlich wird bei den bekannten Elektortherapei-Geräten von zwei Reizstrom-Methoden ausgegangen, dem polaritätsabhängigen "Polaritären Reizprinzip" und dem polaritätsunabhängigen "Apolaritären Reizprinzip".

Beim "Polaritären Reizprinzip" wird mit niederfrequenten Strömen (Nf-Strom) von 0 - 200Hz gearbeitet. Unter der positiven Elektrode, der Anode, kommt es zur Hyperpolarisation (Erhöhung der Membranspannung), wodurch der Abstand zwischen dem Potential in der Zelle und der Reizschwelle größer wird. Unter der negativen Elektrode, der Kathode, wird die Membranspannung dagegen abgesenkt. Bei Erreichen der Reizschwelle löst die Zelle selbsttätig ein Aktionspotential aus.

Die bekannten Reizstromgeräte arbeiten mit den unterschiedlichen Impulsformen im niederfrequenten Spektrum von ca. > 0 - 200 Hz (Nf-Strom). Es kommen z. B. die sogenannten Dreieckströme, Rechteckströme, Diadynamischen Ströme, Hochvoltströme, Ultrareizströme, faradischen Ströme, um nur einige zu nennen, zur Anwendung. Manche Ströme haben dabei eine Gleichstromkomponente, womit die polaritären Effekte noch unterstützt werden.

Es gibt zwei frequenzabhängige Methoden, Aktionspotentiale therapeutisch zu nutzen:
- Funktionsnachahmungsprinzip:
   Man stellt die Anzanl der Aktionspotentiale fest, die die erregbare Zelle (Nerv oder Muskel) zur Erledigung ihrer Aufgaben erzeugt. In der Therapie werden dann ebenso viele Impulse durch Reizung in der entsprechenden Zelle erzeugt, so daß die Zelle bei der Erfüllung ihrer Aufgaben unterstützt wird.
   So wird z. B. zur Erzeugung von bis zu 6 Einzelkontraktionen in der Sekunde mit einer Frequenz von bis zu 6 Hz gereizt.
- Ermüdungsprinzip:
   Wird die Zelle (Nerv oder Muskel) dagegen gezwungen, durch Reizung mit höherer Frequenz, wesentlich öfter als sie dies zur Erfüllung ihrer Aufgaben machen müßte, Aktionspotentiale zu bilden, ermüdet sie nach kurzer Zeit. Es tritt der entgegengesetzte Effekt ein. Die Ermüdung ist durch energieverbrauchende Prozesse bei der Bildung von Aktions- potentialen zu erklären.
   So kann ein verhärteter Muskel nach diesem Ermüdungsprinzip entspannt werden, indem man ihn mit einer "hohen" Frequenz von z. B. 100 Hz oder 200 Hz reizt.

Um überhaupt Aktionspotentiale zu erzeugen, muß selbstverständlich die Intensität so hoch gewählt werden, daß die Reizschwelle überschritten wird. Die Höhe der einzustellenden Intensität hängt von folgenden Faktoren ab:
- von der Lage (Tiefe) der zu reizenden Zelle im Gewebe (Abstand von der Elektrode),
- von der Größe der Elektroden und
- von den Gewebewiderständen im durchströmten Gebiet, die wiederum von den Parametern der Stromform beeinflußt werden.

In der Praxis sind Stromform und Elektrodengröße vorgegeben. Um nun in einem bestimmten Abstand von der Elektrode (z. B. in der Tiefe des Gewebes) eine Gruppe von Zellen zu reizen, wird die Intensität so lange erhöht, bis es zu Aktionspotentialen kommt. Eventuell nachteilig ist dabei, daß Zellen, die zwischen dem Reizgebiet und der Elektrode liegen und keine höheren Reizschwellen besitzen, schon bei niedrigeren Intensitäten gereizt werden.

Auch bei Zellen, die höhere Reizschwellen besitzen, wird in der Praxis "einfach" (das ist wegen der Hautbelastung nicht immer möglich) die Intensität der Stromimpulse erhöht.

Mit steigender Intensität werden so nacheinander tiefer und tieferliegende Zellen oder Zellen, die immer weiter von den Elektroden entfernt liegen, gereizt.

Beim apolaritären Reizprinzip kommen nur sogenannte mittelfrequente Wechselströme (Mf-Stöme) ohne jedwede Gleichstromkomponente zum Einsatz. Unter Mf-Strömen versteht man sinusförmige Wechselströme mit einer Frequenz von > 1.000 Hz - 100.000 Hz. Eine Einzelschwingung (Wechselimpuls) ist bei ausreichender Intensität polaritär wirksam. Ein aus polaritär an sich unterschwelligen kurzen Wechselimpulsen resultierender Wechselstrom (Mf-Impuls) kann in einer Nerven- oder Muskelzelle ein Aktionspotential auslösen.

Es kommt zu einem "Summationseffekt". Mit steigender Frequenz werden auch immer höhere Intensitäten benötigt, um Aktionspotentiale in den Zellen auslösen zu können. WYSS hat zweifelsfrei bewiesen, daß die Erzeugung von Aktionspotentialen mit Mf-Impulsen völlig unabhängig von polaritären Effekten abläuft. Das bedeutet, daß überall dort, wo die Intensität und die Anzahl der Schwingungen groß genug ist, Aktionspotentiale erzeugt werden, ohne Rücksicht auf die momentane Polarität des Mf-Stromes (WYSS, Oscar A. M.: Prinzipien der elektrischen Reizung. NEUJAHRSBLATT, herausgegeben von der Naturforschenden Gesellschaft in Zürich auf das Jahr 1976, Kommissionsverlag Leemann AG Zürich, 1976,28 - 34).

Es werden Mf-Impulse im Nf-Rhythmus von > 0 - ca. 200 Hz und Mf-Trägerfrequenzen von > 1.000Hz - 100.000Hz eingesetzt. In der Praxis ist dies meist ein sinusförmig amplitudenmodulierter Mf-Strom (AM-Mf-Strom) Die folgenden Prinzipien stimmen mit den in Verbindung mit dem "Polaritären Reizprinzip" beschriebenen überein.
- Funktionsnachahmungsprinzip:
   Synchron zu den Mf-Impulsen (AM's-Frequenz) kommt es in erregbaren Zellen zu Aktionspotentialen. Die Zelle wird damit angehalten, ihre natürlichen Funktionen auszuüben, die sich auf Grund dieser Frequenz ergeben.
- Ermüdungsprinzip:
   Um erregbare Zellen zu ermüden, kommen Mf-Impulse mit höheren AM's-Frequenzen zum Einsatz.

Mit steigender Stromintensität werden nacheinander tiefer und tiefergelegene (weiter weg von den Elektroden) Zellen gereizt.

Mit höher werdender Mf-Trägerfrequenz wird immer mehr Intensität zur Erzeugung von Aktionspotentialen benötigt (WYSS, a. a. O., 41 - 43, Abb. 17 / S. 41, Abb. 18 / S. 42). Die Abbildungen sind mit freundlicher Genehmigung von Professor Dr. Oscar A. M. WYSS aus dem Büchlein "Prinzipien der elektrischen Reizung" entnommen. Die Abb. 17 und 18 zeigen die Abhängigkeit der Reizschwellen von Mittelfrequenz-Impulsen als Funktion der Trägerfrequenz .

Auf der Grundlage der mittelfrequenten Wechselströme sind folgende zusätzlichen Tehrapiemöglichkeiten gegeben:
Wird mit (amplitudenkonstantem) Mf-Strom ausreichender Intensität gereizt, so entsteht zunächst ein Aktionspotential. Bei Mf-Strom, der längere Zeit fließt, bleibt der abfallende Schenkel des Aktionspotentials auf einem Depolarisationsniveau (Dauerdepolarisation) stehen, das etwa die Hälfte der Ruhespannung ausmacht. Nach Ausschalten des Mf-Stromes fällt die Membranspannung dann verzögert auf das Niveau der Ruhespannung ab (WYSS, a.a.O., Abb. 13 / S. 36). Die folgenden Unterpunkte beschreiben die therapeutische Nutzung der Dauerdepolarisation.
- Blockierung
   -- Schmerzlinderung und Beeinflussung der Durchblutung
      Bei hohen Intensitäten, die je nach Beschaffenheit des Behandlungsgebietes an der Verträglichkeitsgrenze liegen, kommt es durch die Dauerpolarisation zur Blockierung der Nervenübertragungswege. Diese echte Blockierung der Nerven (der Nachweis wurde von BOWMAN, Bruce R.,1981, Dissertation E. K. University of Ljubljana, Rancho Los Amigos Hospital, Downey, California USA, geliefert) wird z. B. zur Schmerz-Blockade bei Phantomschmerzen oder zur Stellatum-Blockade bei Durchblutungs- störungen genutzt.
- Muskelkontraktur
   -- Muskeltraining bei Willkürinnervationsschwäche und Muskeldehnung
      Bei intaktem Nerv-Muskel-Apparat wird durch Dauerdepolarisation der gestreifte Muskel (Skelettmuskel) direkt gereizt. Es kommt zur Muskel- kontraktur, die z. B. bei Willkürinnervationsschwäche der Muskulatur oder zur Dehnung der Antagonisten von spastischer Muskulatur genutzt wird. Die Intensität soll während der Behandlung in kurzen Abständen durch Pausen unterbrochen werden. Die Intensität kann auch zwischen 100% und ca.50% des eingestelltenWertes an- und abgeschwellt werden.
   -- Erzeugung starker Muskelkontraktionskräfte
      Es können ohne Ermüdungserscheinungen sehr kräftige Muskelkontraktionen erzeugt werden. Bei tetanischer Kontraktion, die mit Reizstrom von ca. 50 Hz und höher erzeugt werden kann, kommt es dagegen zu einer raschen Abnahme der Muskelkontrakionskraft durch Ermüdung der motorischen Einheiten.
- Zellteilung
   -- Wundheilung und beschleunigte Knochenheilung
      Durch Dauerdepolarisation kommt es in gesunden Zellen zur Zellteilung. So kann z. B. die Wundheilung unterstützt und die Knochenheilung bei Frakturen beschleunigt werden.
      Bei Mf-Strömen kommt es unter Einwirkung des elektrischen Wechselfeldes ferner zur Hin- und Herbewegung (Schütteleffekt) von geladenen Molekülen im durchströmten Gewebe, begleitet von Drehbewegungen der geladenen Molekülanteile. Hierdurch wird eine höhere Wahrscheinlichkeit der "richtigen" Begegnungsposition von Enzym und Substrat erreicht, die bei Stoffwechselprozessen chemisch miteinander reagieren (Stoffwechselerleichterung). Der Schütteleffekt wirkt konzentrations- unterschiedsausgleichend, indem Diffusionsprozesse, die auf Grund bestehender Konzentrationsgradienten in bestimmten Richtungen bevorzugt ablaufen, durch die zusätzlich mitgeteilte kinetische Energie beschleunigt werden (Mf-Iontophorese, Entzündungshemmung, Schmerzlinderung).

Der Schütteleffekt ist bei hohen Intensitäten besonders wirksam.
- Verteilung von Entzündungs- und Schmerzmediatoren
- Entzündungshemmung und Schmerzlinderung
   Bei schmerzhaften, entzündlichen Prozessen besteht im kranken Gewebe regelmäßig eine hohe Konzentration von Entzündungs- und Schmerzmediatoren. Durch den Schütteleffekt wird diese hohe Konzentration abgebaut (verteilt). Die "Schüttelintensität", durch hohe Stromintensitäten hervorgerufen, ist ebenso wie die Frequenz für die therapeutischen Wirkungen von großer Bedeutung (HANSJÜRGENS, MAY, Elektrische Differential-Therapie, Karlsruhe 1990).
- Stoffwechselbeeinflussung (Diffusion, Mitochondrien, cAMP)
- Stoffwechselerleichterung und Stoffwechselförderung
Wie oben beschrieben, werden die biochemisch ablaufenden Stoffwechselvorgänge erleichtert.

Auch ist bei Durchstömung von Zellkulturen mit Mf-Strom festgestellt worden, daß die Anzahl der Mitochondrien ("Energiekraftwerke" der Zellen) und deren Größe signifikant zugenommen hat (KOMITOWSKI und EHEMANN 1990, interne Mitteilung).

Ein wichtiger Botenstoff der Zelle, das cAMP, kann ebenfalls durch Mf-Strom in seiner Konzentration beeinflußt werden. Es besteht eine Mf-Intensitätsabhängigkeit (DERTINGER, 1989, Kernforschungszentrum Karlsruhe, NAGY, Nemectron GmbH Karlsruhe).

Mit Mf-Strömen kann darüberhinaus eine sohmerzfreie kräftige Muskelkon- traktion in Form von Muskelkontraktur generiert werden.

Ab 8 kHz kommt es zur sogenannten "Schwellendissoziation", d. h. die Schwellenstromstärke für Muskelkontraktionen tritt unter die der sensiblen Scholle (EDEL, H.: Fibel der Elektrodiagnostik und Elektrotherapie, Müller & Steinicke München 1983, S. 193). Es können kräftigste Muskelkontraktionen ohne Schmerzen ausgelöst werden. Therapeutisch gesehen ist die Schwellendissoziation besonders interessant bei Ausnutzung des reversiblen Vorgangs der Muskelkontraktur, die durch die Dauerdepolarisation des Mf-Stromes hervorgerufen wird.

Durch hohe Intensitäten des Mf-Stromes wird im durchströmten Gewebe Wärme erzeugt. Vorbedingung ist jedoch, daß es nicht zu Belästigungen des Patienten durch Überschreitung der Schwellen (Empfinden, Muskel, Verträglichkeit, Schmerz) kommt.

Anolog zur Verbesserung der Stoffwechselprozesse kann mit Mf-Strom auch eine Iontophorese erreicht werden, also ein Einbringen von Medikamenten mit Hilfe des Stromes durch die Haut in den Körper. Auf Grund der physikalischen Gegebenheiten benötigt man für eine Iontophorese mit Mf-Strom im Vergleich zum galvanischen Strom eine längere Behandlungszeit und höhere Intensitäten.

Wie vorstehend beschrieben und insoweit auch in der einschlägigen Fachliteratur nachzulesen (vgl. Buch "EDiT ® - Elektrische Differential-Therapie" von A. HANSJÜRGENS und H. U. MAY, Ⓒ 1990, Nemectron GmbH, Karlsruhe) arbeiten die bekannten Elektrotherapie-Geräte je nach Befund mit niederfrequenten Strömen bzw. mit amplitudenmodulierten mittelfrequentenStrömen mit Frequenzen von > 0 - 200Hz oder mit mittelfrequenten Strömen mit einer Frequenz von 1.000Hz bis 100.000Hz mit jeweils konstanter Amplitude (Intensität).

Die der vorliegenden Erfindung zugrunde liegende Aufgabe besteht darin, ein elektrotherapeutisches Gerät der gattungsgemäßen Art anzugeben, mit dem gleichermaßen und insoweit synergistisch die mit nieder- und mittelfrequenten Strömen erzielbaren therapeutischen Wirkungen erreicht werden.

Diese Aufgabe wird erfindungsgemäß dadurch gelöst, daß die Arbeitsfrequenz in einem Frequenzband von bis zu mehreren kHz mit einer Periodizität im NF-Bereich von > 0 bis ca. 200Hz frequenzmoduliert ist.

Mit anderen als im Patentanspruch benutzten Worten besteht die technische Lehre des erfindungsgemäßen elektrotherapeutischen Gerätes darin, einen dem medizinischen Befund und der konjugierten Elektrotherapie entsprechenden mittelfrequenten Strom auszuwählen und die Frequenz des mittelfrequenten Stromes in einem Frequenzband von beispielsweise 2.000Hz mit einer Modulationsfrequenz von > 0 - 200Hz, d. h. mit einer Frequenz der NF-Ströme, periodisch zu variieren. Das erfindungsgemäße Gerät arbeitet somit nach den Gesetzen der Frequenzmodulation (und nicht wie aus dem Stand der Technik bekannt, nach den Gesetzen der Amplitudenmodulation).

Die Mf-Ströme mit konstanter Amplitude (Intensität) werden mithin zur Erzeugung von Aktionspotentialen im niederfrequenten Rhythmus (NFR) eingesetzt.

Demgegenüber wird bei allen bekannten Reizstrom-Methoden zur Erzeugung von Aktionspotentialen die Intensität des Reizstromes im niederfrequenten Rhythmus (NFR > 0 - ca. 200 Hz) auf- und abgeregelt. Die Höhe der Intensität der jeweiligen Impulse (Nf-Impuls oder Hüllkurve des Mf-Impulses) richtet sich nach der Reizschwelle der zu reizenden Zelle und nah dem Abstand der Zelle zur Elektrode. Bei tieferliegenden Zellen wird eine höhere Intensität benötigt, um den Spannungsabfall auszugleichen, der an dem Gewebe zwischen Elektrode und den zu reizenden Zellen entsteht. Die Kurve der Reizschwelle wird bei jedem Impuls bei aufsteigender und bei abfallender Intensität (vertikal) durchfahren.

Der elektrophysiologische Hintergrund der erfindungsgemäßen Lehre ist wie folgt:
Die Abhängigkeit der Reizschwelle von der Intensität und der Frequenz wurde bereits beschrieben. Je höher die Frequenz des Stromes, desto größer muß seine Intensität sein, um die Reizschwelle zu überschreiten. Diese linear ansteigenden Kurven elektro- physiologischer Schwellen können nun nicht nur durch Veränderung der Stromintensität, sondern auch durch Variation der Frequenz über- oder unterschritten werden.

Bei der erfindungsgemäßen Methode der Frequenzmodulation werden nun ausschließlich Mf-Ströme eingesetzt. Die Intensität wird während der Behandlung konstant gehalten, nachdem sie auf den gewünschten Wert eingestellt wurde. Die Trägerfrequenz des amplitudenkonstanten Mf-Stromes wird im NFR moduliert (FM-MF-Strom), wobei die Kurve der Reizschwelle bei abfallender und wieder aufsteigender Frequenz horizontal durchfahren wird. Es kommt somit synchron zum NFR zur Erzeugung von Aktionspotentialen.

Weiterbildungen und besondere Ausgestaltungen des erfindungsgemäßen Grundprinzipes sind Gegenstand der Unteransprüche.

Die Einzelheiten werden wie folgt anhand der weiteren Zeichnungen näher erläutert. Diese zeigen in
- Fig. 1: eine Prinzipdarstellung der Reizung mit frequenzmoduliertem mittelfrequentem Strom;
- Fig. 2: einen Ausschnitt aus Fig. 1 zur Erläuterung eines Frequenzfensters im Strom-Frequenz-Diagramm;
- Fig. 3: den Ausschnitt nach Fig. 2 im Frequenz-Zeit-Diagramm;
- Fig. 4: eine Darstellung eines frequenzmodulierten Mf-Stromes;
- Fig. 5: eine Variation des Frequenzfensters nach Fig. 3;
- Fig. 6: eine Prinzipdarstellung der Reizung (Interferenz) mit amplitudenmoduliertem mittelfrequentem Strom;
- Fig. 7: eine Darstellung nach Fig. 6 mit veränderlicher Reizfrequenz;
- Fig. 8: eine Darstellung nach Fig. 1 mit 2 sich überlagernden frequenzmodulierten mittelfrequenten Strömen;
- Fig. 9: ein Blockschaltbild eines erfindungsgemäßen Gerätes;
- Fig. 10: ein Schalttableau des Gerätes nach Fig. 9;
- Fig. 11: eine zweipolige Elektrodenanlage;
- Fig. 12: ein Aktionspotential.

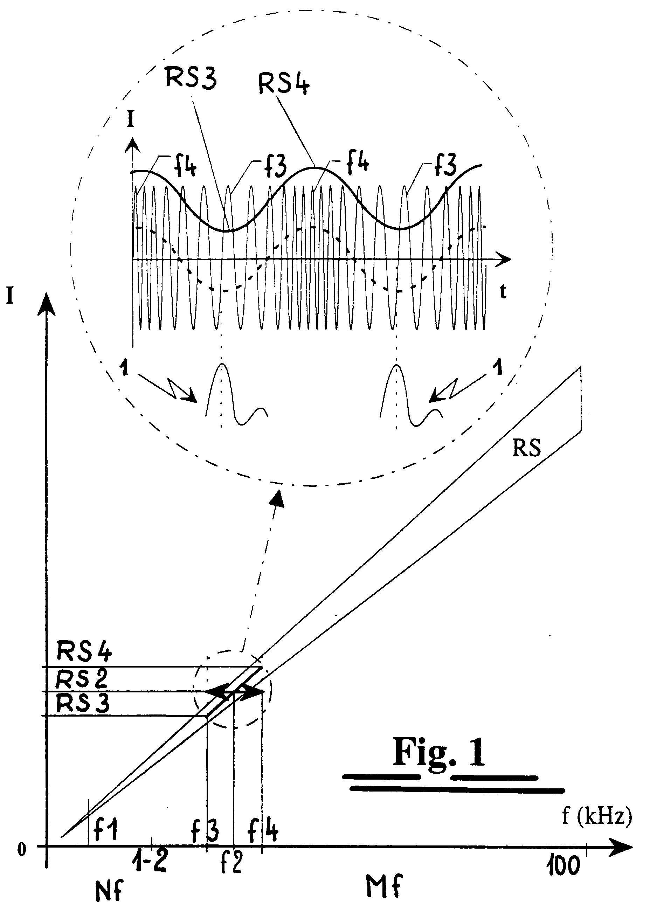

Fig. 1 zeigt im Strom(I) - Frequenz(f) - Diagramm die bekannte Darstellung einer Reizschwelle RS mit dem vom Nf-Bereich aus divergierenden Streubereich (vgl. WYSS a.a.O. Abb. 18).

In Fig. 1 wird die Frequenz des Mf-Stromes zwischen Werten f3 (tiefste Frequenz) und f4 (höchste Frequenz) im niederfrequenten Rhythmus (NFR) moduliert. f3 und f4 sollen als Eckfrequenzen des sich ergebenden Frequenzfensters bezeichnet werden.

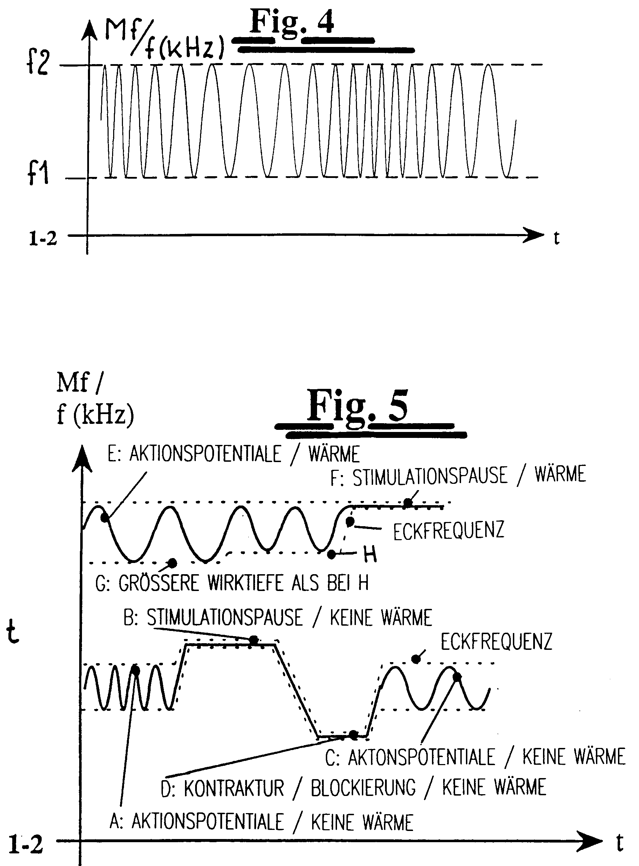

Der Bereich des gesamten Frequenzfensters ist im oberen Teil von Fig. 1 vergrößert abgebildet, wobei der Verlauf des FM-Mf-Stromes ebenso wie der der Reizschwelle und der der Frequenzmodulationskurve (FM-Kurve) jedoch zeitlich dargestellt sind. Die Frequenz der FM-Kurve sei f1, hat also einen Wert aus dem Nf-Bereich. (Ein Beispiel einer frequenzmodulierten FM-Kurve ist in Fig. 4 dargestellt).

Für z. B. f1=2 Hz werden je Sekunde zwei Aktionspotentiale 1 erzeugt, da die Reizschwelle RS3 jedesmal bei Erreichen der Frequenz f3 mit der eingestellten Intensität des Mf-Stromes sicher überschritten wird. Im Zeitpunkt von f4 ist die konstant gehaltene Intensität des Mf-Stromes nicht mehr groß genug, um die Reizschwelle RS4 zu erreichen.

Um Aktionspotentiale mit FM-Mf-Strömen im NFR erzeugen zu können, müssen folgende voneinander abhängige Parameter richtig eingestellt werden:
- die Trägerfrequenz des Mf-Stromes,
- die Amplitude des Mf-Stromes,
- die Modulationsfrequenz und
- die Eckfrequenzen der Frequenzmodulation (Frequenzfenster).

Nach den jeweiligen therapeutischen Erfordernissen, die bei Anwendung von FM-Mf-Strömen sowohl die Nutzung von Aktionspotentialen als auch die Wirkungen des Mf-Stromes erlauben, wird die Trägerfrequenz des Mf-Stromes gewählt. So können die folgenden Wirkungen des Mf-Stromes gleichzeitig mit denen der Aktionspotentiale genutzt werden: Wärmeerzeugung, Mf-Iontophorese, sowie starke oder schwächere Entzüdungshemmung, Schmerzlinderung und Stoffwechselbeeinflussung.

Mit der Frequenz ergibt sich die einzustellende Intensität des Mf-Stromes (konstante Amplitude). Die Intensität wird so gewählt, daß es noch nicht zur Überschreitung der Schwelle kommt.

Dann wird die Modulationsfrequenz der FM nach den therapeutischen Prinzipien der Funktionsnachahmung oder der Ermüdung eingestellt.

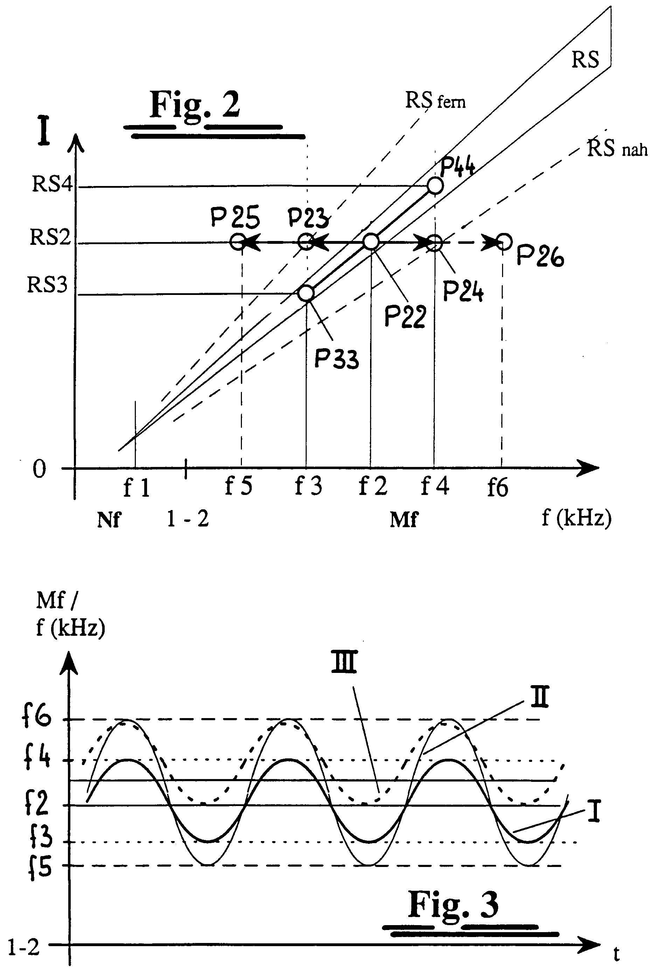

Zum Schluß werden die Eckfrequenzen des Frequenzfensters gewählt, also die Frequenzgrenzen, zwischen denen die Trägerfrequenz sich im NFR verändern soll. Das Frequenzfenster muß mindestens so groß gewählt werden, daß es im Bereich der vorgegebenen Trägerfrequenz sicher zur horizintalen Überschreitung der Schwellenkurve kommt. Die hierzu benötigte Frequenz ist die untere Frequenzgrenze (f3 und Punkt P23, in Fig. 2) des Frequenzfensters; die obere Frequenzgrenze ist die gewählte Trägerfrequenz (f4 und Punkt P24, in Fig. 2).
- Die Vorteile dieser anhand von Fig. 1 (mit Fig. 2 / Fig. 3) dargestellten Methode liegt darin, daß gleichzeitige Therapiewirkungen von Aktionspotentialen und Mf-Strom-Effekten (Schütteleffekt) mit und ohne Wärmeerzeugung möglich sind.

Alle zusätzlichen Therapiemöglichkeiten, die mit amplituden- konstantem Mf-Strom (ak-Mf-Strom) gegeben sind, können gleichzeitig mit den therapeutischen Wirkungen der durch FM-MF-Strom erzeugten Aktionspotentiale und Wärme genutzt werden, und zwar z. B. zur Schmerztherapie mit und ohne Wärme.

Ferner sind neue Therapie-Kombinationen mit und ohne Wärme möglich, und zwar können durch zeitliche und intensitätsmäßige Variation der FM-Kurven neue Kombinationen der Wirkungen des Mf-Stromes (Dauerdepolarisation und Schütteleffekt) und der durch FM-MF-Ströme erzeugten Aktionspotentiale in ein und derselben Behandlung zum Einsatz kommen (vgl. später Fig. 5).

Die Dauerdepolarisation wird eingesetzt:
- zur Blockierung von Zellinformationen - z. B. zur Schmerzblockade bei Phantomschmerzen und zur Stellatum- Blockade bei Durchblutungsstörungen und
- bei gesunden Zellen zur Zellteilung - z. B. bei der Behandlung von Wunden und Frakturen
Der Schütteleffekt wird eingesetzt:
- zur Schmerzmediatorenverteilung,
- zur Verteilung von Entzündungsmediatoren,
- zu konzentrationsausgleichenden Vorgängen zwischen den Zellen,
- zur Beeinflussung von Stoffwechselvorgängen und
- zur Iontophorese mit mittelfrequenten Strömen.

Der Muskelaufbau wird begünstigt, und zwar bei gleichzeitigen Schütteleffekten, ohne therapeutisch nutzbare Wärmeentwicklung.

Dabei liegt die Frequenz f im unteren Mf-Bereich, so daß die benötigte Intensität des Mf-Stromes noch keine Wärme erzeugt.

Unter Hinweis auf Fig. 2 ist noch anzumerken, daß durch intensitätsmäßige Variation der FM-Kurve folgende Effekte erreicht werden können:
- zur Erzeugung von langsamen Wogen der Kontraktur (Erfassung von tieferen und nicht so tiefliegenden motorischen Einheiten) kann die Frequenz f der Mf-Ströme in mehreren Sekunden von f5 (Punkt P25) nach f3 (Punkt P23) und wieder zurück nach f5 kontinuierlich verändert werden.
- zur Erzeugung von längeren Behandlungsphasen zwischen den Aktionspotentialen, in denen sich die Zelle erholen kann und nur der Schütteleffekt erzeugt wird, kann die Frequenz f der Mf-Ströme von f3 (Punkt P23) nach f6 (Punkt P26) verändert werden und nicht nur von f3 (Punkz P23) nach f4 (Punkt P24), wie es zur Erzeugung von Aktionspotentialen notwendig ist.

Fig. 3 zeigt mehrere FM-Kurven, bei denen sowohl die Arbeitsfrequenz f (vgl. FM-Kurven I+II mit III), als auch die Eckfrequenzen (vgl. FM-Kurve I mit II) variiert werden. Die Variation der Eckfrequenzen hat die in Fig. 2 beschriebenen Effekte, wahrend die Veränderung der Arbeitsfrequenz durch die höhere Stromintensität (vgl. in Fig. 2 Punkt P24 mit Punkt P44 bei F4) zusätzlich Wärme erzeugt.

Der Muskelaufbau wird durch gleichzeitige Wärmeerzeugung durch Wahl einer höheren Arbeitsfrequenz f der Mf zusätzlich gefördert.

Durch die erfindungsgemäße Anwendung von amplitudenkonstanten mittelfrequenten Strömen kommt es zu weiteren elektrophysiologisch bedingten Vorteilendieser Methode bei der Reizung:
- keine NF-Reizung der Haut durch Intensitätsveränderungen des Reizstromes, dadurch schmerzlose Anwendung;
- gebündeltes Eindringen der Stromlinien senkrecht zu den Hautschichten, dadurch nur geringe Energieverluste des Stromes bei der Überwindung der Hautschranke und hohe Reizwirkung subcutan und in der Tiefe;
- Ausnutzung der geringen Hautwiderstände des Mf-Stromes (mit steigender Frequenz nimmt der Hautwiderstand ab), dadurch schmerzlose Anwendung und nur geringe Energieverluste des Stromes bei der Überwindung der Hautschranke;
- nachlassendes Stromgefühl nach wenigen Minuten der Behandlung durch Dauerdepolarisierung des Mf-Stromes.

Anhand von Fig. 5 sei gezeigt, wie z. B. durch zeitliche Variationen der FM-Kurve die folgenden Wirkungen nacheinander hervorgerufen werden können (f_{FM-K} hat hierbei die Bedeutung einer einstellbaren Festfrequenz):
- tetanische Kontraktionen (f_{FM-K} >20 Hz),
- Pause (f_{FM-K} = 0, f von Mf= obere Eckfrequenz),
- Kontraktur (f von Mf= untere Eckfrequenz ),
- Pause (f_{FM-K} = 0, f von Mf= obere Eckfrequenz) und wieder
- tetanische Kontraktionen (f_{FM-K} >20 Hz) usw..

Im oberen Teil der Darstellung nach Fig. 5 ist eine Zwischen den Eckfrequenzen f3 und f4 variierte FM-Kurve gezeigt. Im Kurven- bereich E werden Aktionspotentiale generiert, und gleichzeitig entsteht Wärmewirkung. Nach einer vorgegebenen Zeitspanne wird die Eckfrequenz f3insoweit verändert, als - entsprechend der gegenüber G variierten Kurze H - die Wirktiefe verringert wird. Im weiteren zeitlichen Verlauf wird bei konstantem Strom nur noch Wärme erzeugt (vgl. F).

Im unteren Teil der Darstellung nach Fig. 5 ist eine weitere Behandlungskurve gezeigt. Zunaächst wird eine FM-Kurve mit den Eckfrequenzen f1 und f2 eingestellt, die Aktionspotentiale generiert; auf Grund der tieferen Frequenzen wird - anders als in der oberen Kurve - keine Wärme erzeugt (vgl. A). Die FM-Kurve wird von einer Stimulationspause abgelöst (vgl. B) und zwar entsprechend einem über der Eckfrequenz f2 liegendem Niveau; die benötigte Stromstärke reicht nicht zur Wärmeerzeugung aus. Anschließend wird zur Erzeugung einer Kontraktur und/oder Blockierung ein Strom mit einer Frequenz angelegt, die tiefer als die untere Eckfrequenz f1 ist (vgl. D). Danach werden wiederum Aktionspotentiale generiert (vgl. C) und zwar ebenfalls ohne Wärmeerzeugung.

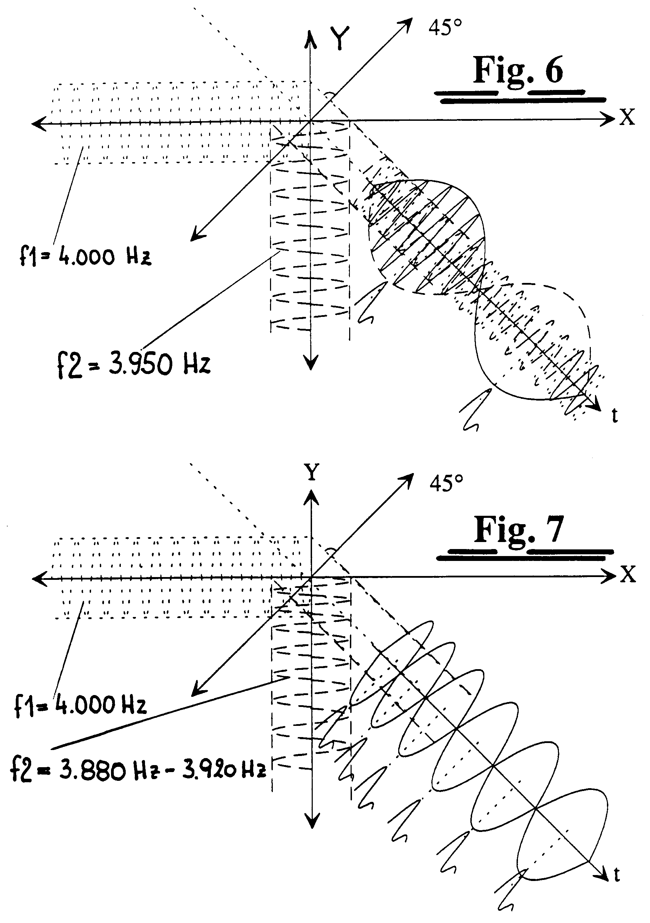

Anhand der Fig. 1...5 wurde die Generierung von Aktionspotentialen auf Grund der erfindungsgemäß frequenzmodulierten Arbeitsfrequenz gezeigt. Über die Fig. 6, 7 und 8 sollen Weiterbildungen in dem Sinne offenbar werden, daß zwei Mf-Ströme zur Überlagerung gebracht werden (Interferenz-Methode). Die Überlagerung zweier mittelfrequenter Ströme geringer Frequenzabweichung zur Erzeugung niederfrequenter Interferenzströme ist bei Therapiegeräten an sich z.B. aus DE-A-33 35 849 bekannt. Dabei kommt es im Überlagerungsfeld beider Mf-Ströme zur Amplitudenmodulation (AM).

Es entstehen also amplitudenmodulierte Ströme wobei die genauen Vorgänge in Fig. 6 und 7 gezeigt sind. Die AM entsteht durch die Frequenzdifferenz der beiden Mf-Ströme. Ein Strom hat dabei eine feste Mittelfrequenz von z . B. 4.000 Hz , der andere Stromkreis hat eine feste Frequenz, die z. B. zwischen 3.800 Hz und 4.000 Hz eingestellt werden kann. In Gebieten, in denen sich die beiden Ströme überlagern, kommt es zur Interferenz. Hat der Stromkreis mit der einstellbaren Frequenz z. B. eine Frequenz von 3.950 Hz, so entsteht ein amplitudenmodulierter mittelfrequenter Strom, dessen Amplitude mit 50 Hz moduliert ist (vgl. Fig. 6). Bereiche zwischen 3.800Hz und 4000 Hz können zusätzlich mit einer sehr langsamen Frequenz <0 - ca 0,1 Hz moduliert werden. Dies geschieht hierbei jedoch nicht zum Zwecke der Erzeugung von Aktionspotentialen und ist nicht mit der FM der vorliedenden Erfindung zu verwechseln. Schließlich ist weder die Modulationsfrequenz zwischen <0 - 200 Hz, noch ist das Frequenzfenster von 200 Hz ausreichend, um überhaupt Aktionspotentiale zu erzeugen.

Fig. 7 zeigt ein Beispiel mit einer Modulationsfrequenz von 1/15Hz und einem Frequenzbereich von 80Hz - 120Hz dargestellt. Es werden also 80 Aktionspotentiale erzeugt, die sich in 15 Sekunden kontinuierlich auf 120 erhöhen.

Die AM erfolgt beim Interferenzverfahren in den beiden Richtungen der 45°-Linie und mit einer Phasenverschiebung von 90° in den beiden Richtungen, die durch eine Linie, senkrecht zur 45°-Linie (vgl. Fig 6,7, gestrichelte Linie) gekennzeichnet ist.

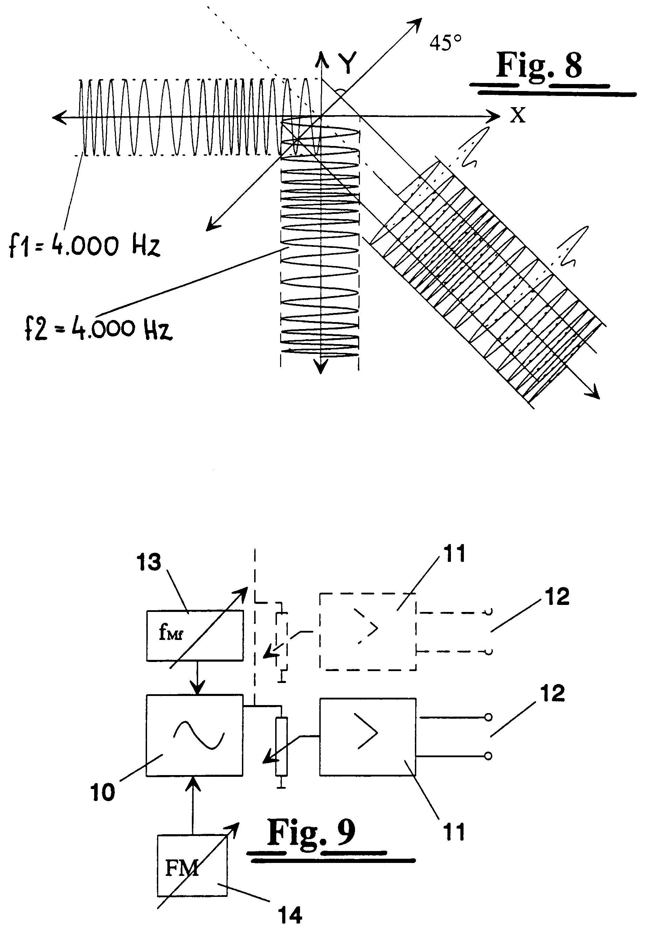

Fig. 8 zeigt den resultierenden Strom einer FM-Mf-Reizung bei Überlagerung von zwei Strömen. Das Ziel einer solchen Überlagerung von zwei oder mehr Stromkreisen ist es, die Intensität im Überlagerungsgebiet (Behandlungsgebiet) durch Addition der Einzelintensitäten soweit zu erhöhen, daß es hier zur Auslösung von Aktionspotentialen und zur Wärmeentstehung kommt.

Ist der Phasenunterschied beider Ströme =0, kommt es nur in den Richtungen der 45°-Linie (vgl. Fig. 8) zur Intensitätserhöhung. Wird dagegen die Phase mit einer Periodizität von >0 bis etwa 0,1 Hz um 180° gedreht, so entsteht die Erhöhung der Intensität abwechselnd in den 45°-Richtungen und in den beiden Richtungen sank recht zur 45°-Linie (vgl. Fig. 8, gestrichelte Linie).

Auch können die Frequenzen beider Ströme einen unterschiedlichen Wert aufweisen, so daß es wie in Fig. 6 und 7 gezeigt, im Überlagerungsgebiet zur AM kommt. Durch langsame FM der erfindungsgemäßen FM-Mf-Reizung kommt es bei f3 (vgl. Fig. 1) zur Auslösung von Aktionspotentialen durch die AM, bei f4 wirkt dann nur noch die Mf Schütteleffekt und gegebenenfalls Wärme.

Fig. 9 zeigt ein Blockschaltbild eines erfindungsgemäß arbeitenden elektotherapeutischen Gerätes für einen und auch für zwei Stromkreise.

Das in Fig. 9 dargestellte elektrotherapeutische Gerät besteht im Prinzip aus einem Oszillator 10, an dem parallel ein (oder mehrere) Verstärker 11 angekoppelt ist (sind). Jedem Verstärker 11 ist ein Patientenanschluß 12 zugeordned, über den - diametral zueinander - Elektrodenanschlüsse an zu behandelnde Körperteile angelegt werden. Der Oszillator 10 ist mit einem Frequenzgenerator 13 verbunden, der die eigentliche Arbeitsfrequenz f_{Mf} bestimmt;dem Oszillator 10 ist weiterhin ein Frequenzmodulator 14 zugeordnet, über den die Arbeitsfrequenz f_{Mf} innerhalb der vorgegebenen Eckfrequenzen moduliert wird.

Fig. 10 zeigt ein Schalttableau eines erfindungsgemäß- elektrotherapeutischen Gerätes, wobei dieses Gerät als mittels Bedienungsknöpfen einfach und als tragbares Heimgerät auch von Laien bedienbares Gerät dargestellt ist. Je nach Therapie sind die unterschiedlichen Stromstärken und Frequenzen einstellbar und es ist gleichermaßen denkbar, dieses Gerät mit einem Steuerungsmodul (Mikroprozessor) zu verbinden, das entsprechend den in Verbindung mit Fig. 5 aufgezeigten Möglichkeiten spezifische Behandlungs- programme initiiert.

In Fig. 11 ist als Anwendungsbeispiel eines gemäß den erläuterten Vorgaben und Randbedingungen arbeitenden elektrotherapeu- tischen Gerätes eine 2-polige Elektrodenanlage dargestellt.

Um die beabsichtigte Wirkung in der Nähe der Elektroden 20.1, 20.2 erzielen zu können, muß sichergestellt werden, daß im Behandlungsgebiet 21 die Stromdichte, also die Stromstärke pro Flächeneinheit, ausreichend hoch ist. Dies wird entsprechend der dargestellten Anlage mit einem über die zwei Elektroden und durch das Behandlungsgebiet geschlossenen Stromkreis erreicht. Die höchsten Stromdichten und die damit verbundenen therapeutischen Wirkungen entstehen dabei jeweils in Elektrodennähe, d. h. im Behandlungsgebiet (vgl. die in Fig. 11 gepunktet hervorgehobenen Gebiete).

Ist das Behandlungsgebiet in der Tiefe, so werden - vgl. Fig. 6, 7 und 8 - vier Elektroden, d. h. zwei Stromkreise so angelegt, daß es zu einem Überlagerungsfeld in der Tiefe des Gewebes kommt, also im Behandlungsgebiet. In diesem Gebiet wird die Intensität durch Addition der Intensitäten beider Stromkreise gezielt erhöht.

## Patentansprüche

1. Im Mittelfrequenzbereich zwischen 1.000 Hz und 100.000 Hz arbeitendes Gerät für elektrotherapeutische Anwendungen, wobei bezogen auf ein zu behande indes Körperteil ein Stromkreis mit einem mittelfrequenten Strom (Mf-Strom) über zwei Elektroden angelegt wird,
dadurch gekennzeichnet,
daß die Amplitude des MF-Stroms konstant gehalten ist und die Frequenz um tausend bis mehrere tausend Hz (Eckfrequenzen) mit einer Modulationsfrequenz von > 0 bis einigen hundert Hz (z.B. 200 Hz) moduliert ist, um synchron zur Modulationsfrequenz Aktionspotentiale im Behandlungsgebiet zu erzeugen.

2. Elektrotherapeutisches Gerät nach Anspruch 1,
dadurch gekennzeichnet,
daß die Frequenz des MF-Stroms so hoch eingestellt ist, daß über die zur Erreichung einer Reizschwelle dadurch notwendige höhere Intensität eine zusätzliche Wärmeerzeugung entsteht.

3. Elektrotherapeutisches Gerät nach Anspruch 1 oder 2,
dadurch gekennzeichnet,
daß die Eckfrequenzen und/oder die Modulationsfrequenz über die Behandlungszeit betrachtet variierbar sind.

4. Elektrotherapeutisches Gerät nach einem der Ansprüche 1 bis 3,
dadurch gekennzeichnet,
daß die Modulationsfrequenz in einem Bereich von > 0 Hz bis etwa 0,1 Hz moduliert wird.

5. Elektrotherapeutisches Gerät nach einem der Ansprüche 1 bis 4,
dadurch gekennzeichnet,
daß zwei oder mehrere Stromkreise über je zwei Elektroden in der Weise angelegt werden, daß sich die Ströme im Behandlungsgebiet kreuzen, wobei die Frequenzen der Ströme gleich sind oder sich um einen Betrag zwischen > 0 bis etwa 200 Hz unterscheiden.

6. Elektrotherapeutisches Gerät nach Anspruch 5,
dadurch gekennzeichnet,
daß zwischen den bei den Strömen ein Phasenunterschied mit konstantem Wert oder im Bereich von >0 bis etwa 0,1 Hz moduliert besteht.

7. Elektrotherapeutisches Gerät nach einem der Ansprüche 1 bis 6,
gekennzeichnet durch seine Ausbildung als Heimgerät.

## Claims

1. Apparatus for electrotherapeutic applications operating in the medium-frequency range between 1000 Hz and 100,000 Hz where, in relation to a body part to be treated, a circuit with a medium-frequency current (MF current) is applied across two electrodes, characterized in that the amplitude of the MF current is kept constant and the frequency is modulated by one thousand to several thousand Hz (corner frequencies) with a modulation frequency of > 0 to several hundred Hz (for instance 200 Hz) in order to generate in synchronism with the modulation frequency action potentials in the treatment area.

2. Electrotherapeutic apparatus according to claim 1, characterized in that the frequency of the medium-frequency current is adjusted to a level such that through the elevated intensity necessary for obtaining a stimulus threshold an additional generation of heat is induced.

3. Electrotherapeutic apparatus according to claim 1 or 2, characterized in that the corner frequencies and/or the modulation frequency, viewed over the treatment time, are variable.

4. Electrotherapeutic apparatus according to one of the claims 1 through 3, characterized in that the modulation frequency is modulated in a range of > 0 to about 0.1 Hz.

5. Electrotherapeutic apparatus according to one of the claims 1 through 4, characterized in that two or several circuits are applied by way of two electrodes each, in such a way that the currents intersect in the treatment area, the frequency of the currents being equal or differing by an amount between > 0 to about 200 Hz.

6. Electrotherapeutic apparatus according to claim 5, characterized in that a phase difference with a constant value or modulated in the range of > 0 to about 0.1 Hz exists between the two currents.

7. Electrotherapeutic apparatus according to one of the claims 1 through 6, characterized by its design as an apparatus for home use.

## Revendications

1. Appareil pour applications électrothérapeutiques travaillant dans la gamme des moyennes fréquences entre 1.000 Hz et 100.000 Hz, par rapport à une certaine partie du corps à traiter, un circuit de moyenne fréquence (courant MF) étant appliqué moyennant deux électrodes,
caractérisé en ce que l'amplitude du courant MF est maintenue constante et que la fréquence est modulée de mille à plusieurs milliers de Hz (fréquences de coupure) par une fréquence de modulation de > 0 à quelques centaines de Hz (par ex. 200 Hz) afin de produire, en même temps que la fréquence de modulation, des potentiels d'action dans le domaine de traitement.

2. Appareil électrothérapeutique selon la revendication 1,
caractérisé en ce que la fréquence du courant MF est réglée à un niveau si élevée que, par l'intensité plus grande nécessaire pour obtenir un seuil de stimulation, il y a une production supplémentaire de chaleur.

3. Appareil électrothérapeutique selon la revendication 1 ou 2,
caractérisé en ce que, considéré pendant toute la durée du traitement, les fréquences de coupure et/ou la fréquence de modulation sont modulables.

4. Appareil électrothérapeutique selon l'une des revendiations 1 à 3,
caractérisé en ce que la fréquence de modulation est modulée dans une gamme de > 0 Hz à env. 0,1 Hz.

5. Appareil électrothérapeutique selon l'une des revendications 1 à 4,
caractérisé en ce que deux ou plusieurs circuits électriques sont appliqués, chacun moyennant deux électrodes, de manière à ce que les circuits se croisent dans le domaine de traitement, les fréquences des circuits étant identiques ou bien diffèrent d'une valeur située entre > 0 et env. 200 Hz.

6. Appareil électrothérapeutique selon la revendication 5,
caractérisé en ce qu'il y a entre les deux circuits une différence de phase de valeur constante ou modulée dans une gamme de > 0 à env. 0,1 Hz.

7. Appareil électrothérapeutique selon l'une des revendications 1 à 6,
caractérisé par sa réalisation comme appareil pour usage domestique.
